# EUROPEAN PATENT APPLICATION

(11) **EP 4 082 615 A1**
(43) Date of publication of application: **02.11.2022**
(21) Application number: 20905606.8
(22) Date of filing: 25.12.2020
(51) Int. Cl.: A61P 15/08, A61P 43/00, C12N 5/073, C12N 5/075, C12N 5/076, C12N 1/00, C12N 1/04, A61K 31/4172

(54) **INHIBITOR OF FERTILIZED EGG FRAGMENTATION**

(30) Priority: 27.12.2019 JP 2019238352
(71) Applicant: National Center for Child Health and Development, Tokyo 157-8535 (JP)
(72) Inventor: MIYADO, Kenji, Tokyo 157-8535 (JP); MIYAMOTO, Yoshitaka, Tokyo 157-8535 (JP)
(74) Representative: Gill Jennings & Every LLP
(86) International application number: PCT/JP2020/048973
(87) International publication number: WO 2021/132655

(57) **Abstract**

Provided is a novel means of inhibiting fertilized egg fragmentation. The present invention provides an inhibitor of fertilized egg fragmentation, said inhibitor comprising selenoneine or ergothioneine, a tautomer or dimer thereof, or a pharmaceutically acceptable salt of the same as an active agent.

## Description

### FIELD

The present invention relates to an agent for inhibiting fragmentation of fertilized egg, and to a method using the agent for inhibiting fragmentation.

### BACKGROUND

Under the recent social trend toward later marriage and older birthing age, a greater number of patients undergoes infertility treatment year by year. There are various causes for infertility, such as lowered sperm count and motility, and lowered egg fertility associated with aging.

Artificial insemination, *in vitro* fertilization and intracytoplasmic sperm injection are known as the types of infertility treatment. Such methods usually use harvested sperm, and often use stored frozen sperm. Sperm are stored at low temperature by refrigeration or freezing after dilution with a diluent in order to lower their motility and metabolism during storage. However, sperm are adversely affected during the freezing process and thawing process, leads to reduced fertilization rates, post-fertilization development rates and implantation rates. In order to alleviate adverse effects during the freezing process and thawing process, protectants are used, and medium components such as buffering agents and saccharides are subjected to refinement. Water-soluble polymer compounds composed of highly polymerized organic polymers are known as protective agents while amino acids are known as auxiliary components, for low temperature storage (see PTL 1: International Patent Publication No. WO2008/007463, for example).

Artificial insemination and *in vitro* fertilization techniques continue to advance year by year, but even with successful fertilization being achieved by such techniques, fertilized eggs do not have a high probability of undergoing proper cell division and developing into viable embryos. The fertilized eggs obtained by *in vitro* fertilization and intracytoplasmic sperm injection often suffer fragmentation by non-uniform splitting during the course of culturing to 4 to 8 cell stages. The cause of such fragmentation is less understood.

Selenoneine is a selenium-containing compound found in fish such as tuna, mackerel and yellowtail, which has a high antioxidant effect and has been shown to exhibit a physiological action such as protection and enhanced proliferation of cultured cells (PTL 2: Japanese Unexamined Patent Publication No. 2019-76039). Ergothioneine, having the selenium of selenoneine replaced by sulfur, is also known to have a high antioxidant effect. Ergothioneine has been reported to have elastase-inhibiting action and tyrosinase-inhibiting action, and is of particular interest in the field of beauty and foods for the purpose of skin whitening and wrinkle prevention.

### [CITATION LIST]

### [PATENT LITERATURE]

[PTL 1] International Patent Publication No. WO2008/007463
[PTL 2] Japanese Unexamined Patent Publication No. 2019-76039

### SUMMARY

### [TECHNICAL PROBLEM]

It is an object of the present invention to provide novel means for inhibiting fragmentation of fertilized eggs.

Among numerous natural substances, the present inventors have focused on selenoneine, an organic selenium compound found in fish, and examined its usefulness in regenerative medicine and reproductive medicine. Then, the inventors have found that selenoneine and ergothioneine, which is similar to selenoneine in terms of its structure, remarkably inhibit fragmentation of fertilized eggs, which leading to the present invention.

Specifically, the present invention encompasses the following inventions:
[1] An agent for inhibiting fragmentation of fertilized eggs, comprising selenoneine or ergothioneine, or a tautomer or dimer thereof, or a pharmaceutically acceptable salt of thereof.
[2] A sperm or egg treatment agent, comprising the agent for inhibiting fragmentation according to [1] above, or selenoneine or ergothioneine, or a tautomer or dimer thereof, or a pharmaceutically acceptable salt thereof.
[3] An embryogenesis-promoting agent, comprising the agent for inhibiting fragmentation according to [1] above, or selenoneine or ergothioneine, or a tautomer or dimer thereof, or a pharmaceutically acceptable salt thereof.
[4] A medium for sperm culturing, egg culturing, fertilized egg culturing or fertilization, comprising the agent for inhibiting fragmentation according to [1] above, or selenoneine or ergothioneine, or a tautomer or dimer thereof, or a pharmaceutically acceptable salt thereof.
[5] A medium for freezing of a sperm, egg or fertilized egg, comprising the agent for inhibiting fragmentation according to [1] above, or selenoneine or ergothioneine, or a tautomer or dimer thereof, or a pharmaceutically acceptable salt thereof.
[6] A sperm culturing method, comprising culturing sperm in medium that includes the agent for inhibiting fragmentation according to [1] above, or selenoneine or ergothioneine, or a tautomer or dimer thereof, or a pharmaceutically acceptable salt thereof.
[7] An egg or fertilized egg culturing method comprising culturing an egg or fertilized egg in medium that includes the agent for inhibiting fragmentation according to [1] above, or selenoneine or ergothioneine, or a tautomer or dimer thereof, or a pharmaceutically acceptable salt thereof.
[8] A method for producing a split embryo, morula or blastocyst, comprising the following steps:
   culturing an egg in medium that includes the agent for inhibiting fragmentation according to [1] above, or selenoneine or ergothioneine, or a tautomer or dimer thereof, or a pharmaceutically acceptable salt thereof,
   fertilizing the cultured egg with sperm, and
   culturing the fertilized egg in fertilized egg culturing medium.
[9] A method for producing a split embryo, morula or blastocyst, comprising the following steps:
   culturing sperm in medium that includes the agent for inhibiting fragmentation according to [1] above, or selenoneine or ergothioneine, or a tautomer or dimer thereof, or a pharmaceutically acceptable salt thereof,
   fertilizing the cultured sperm with an egg, and
   culturing the fertilized egg in fertilized egg culturing medium.
[10] The method according to [8] or [9] above, which further comprises a step of freezing a split embryo, morula or blastocyst in liquid nitrogenization.
[11] The method according to any one of [8] to [10], wherein the step of fertilization is carried out by *in vitro* fertilization or intracytoplasmic sperm injection.
[12] The method according to any one of [8] to [11], wherein the fertilized egg culturing medium further includes the agent for inhibiting fragmentation.
[13] An agent for improving infertility, comprising selenoneine or ergothioneine, or a tautomer or dimer thereof, or a pharmaceutically acceptable salt thereof.
[14] Selenoneine or ergothioneine, or a tautomer or dimer thereof, or a pharmaceutically acceptable salt thereof, for use in infertility treatment.
[15] A method for treating infertility, comprising administering selenoneine or ergothioneine, or a tautomer or dimer thereof, or a pharmaceutically acceptable salt thereof.
[16] Use of selenoneine or ergothioneine, or a tautomer or dimer thereof, or a pharmaceutically acceptable salt thereof, for preparing an agent for improving or treating infertility.

### [ADVANTAGEOUS EFFECTS OF INVENTION]

The present invention makes it possible to inhibit fragmentation of an egg and/or fertilized egg. The present invention also exhibits an effect of protecting sperm.

### BRIEF DESCRIPTION OF DRAWINGS

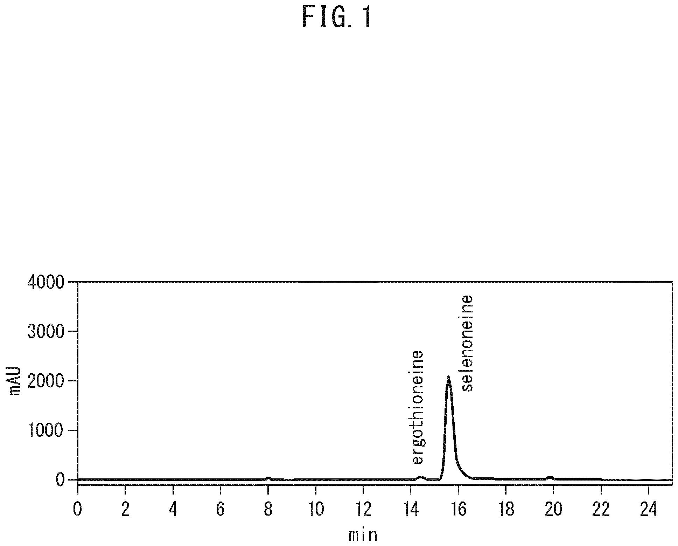

[Fig. 1] Fig. 1 shows the results of analytical HPLC of the 80% ethanol supernatant fraction obtained by purifying partially purified selenoneine by means of preparative HPLC.

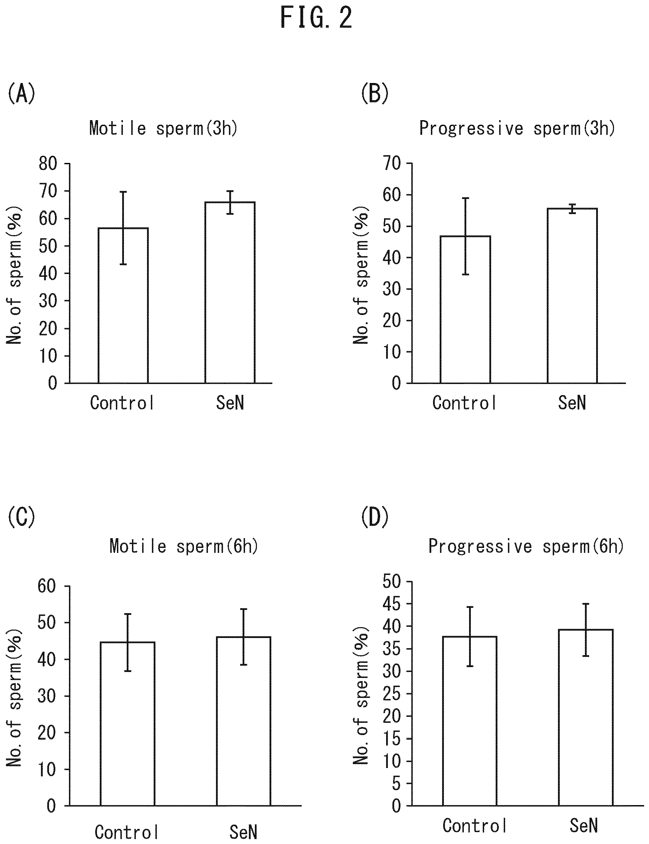

[Fig. 2] Fig. 2 shows the proportion of motile sperm (A) and the proportion of progressive sperm (B) which are cultured for 3 hours, and the proportion of motile sperm (C) and the proportion of progressive sperm (D) which are cultured for 6 hours, in the presence of purified selenoneine (SeN) or in its absence (Control).

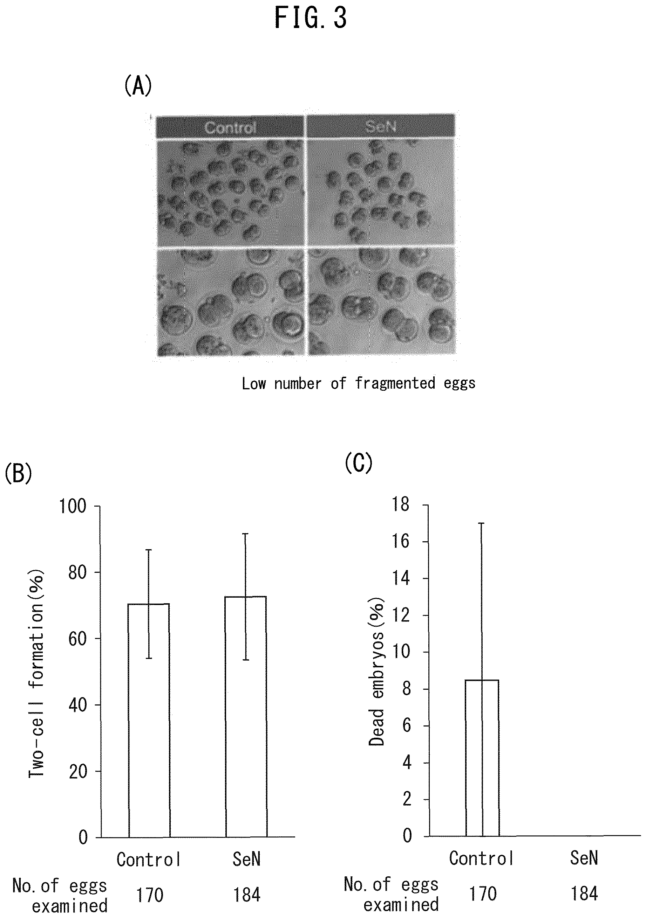

[Fig. 3] Fig. 3A shows a photomicrograph of fertilized eggs cultured in the presence of purified selenoneine (SeN) or in its absence (Control). Fig. 3B shows the proportion of 2-cell formation of fertilized eggs cultured in the presence of purified selenoneine (SeN) or in its absence (Control), and Fig. 3C shows the proportion of fragmented fertilized eggs among fertilized eggs cultured in the presence of purified selenoneine (SeN) or in its absence (Control).

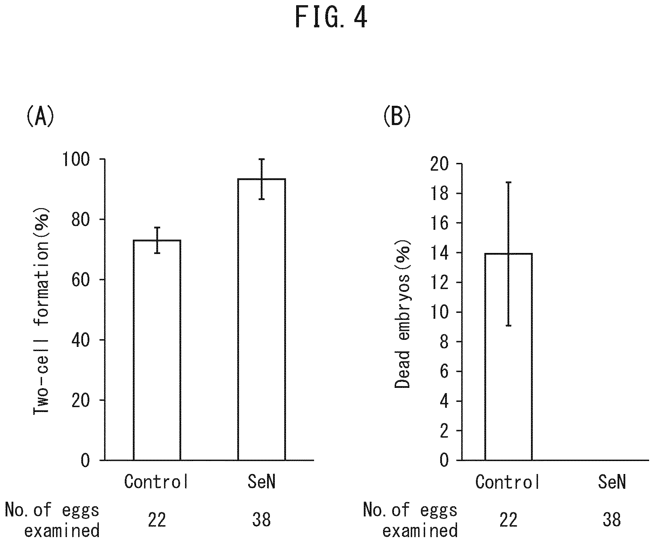

[Fig. 4] Fig. 4A shows the proportion of 2-cell formation of fertilized eggs which have been fertilized with sperm cultured for 6 hours in the presence of purified selenoneine (SeN) or in its absence (Control), and Fig. 4B shows the proportion of fragmented fertilized eggs among fertilized eggs which has been fertilized with sperm cultured for 6 hours in the presence of purified selenoneine (SeN) or in its absence (Control).

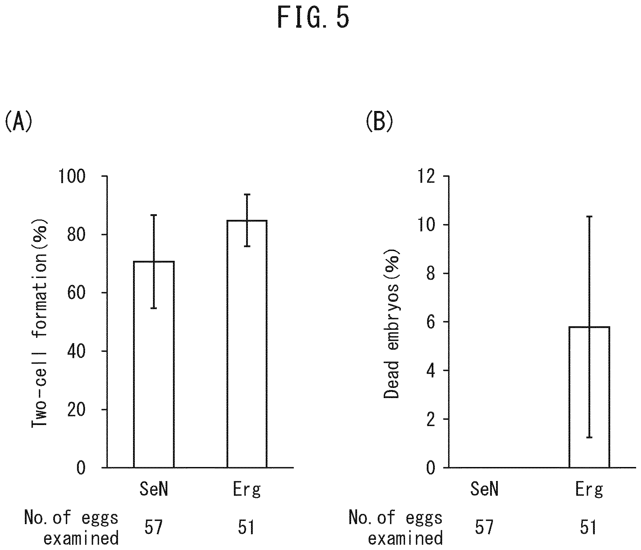

[Fig. 5] Fig. 5A shows the proportion of 2-cell formation of eggs which have been fertilized with sperm cultured for 6 hours in the presence of purified selenoneine (SeN) or purified ergothioneine (Erg), and Fig. 5B shows the proportion of fragmented eggs among eggs which have been fertilized with sperm cultured for 6 hours in the presence of purified selenoneine (SeN) or purified ergothioneine (Erg).

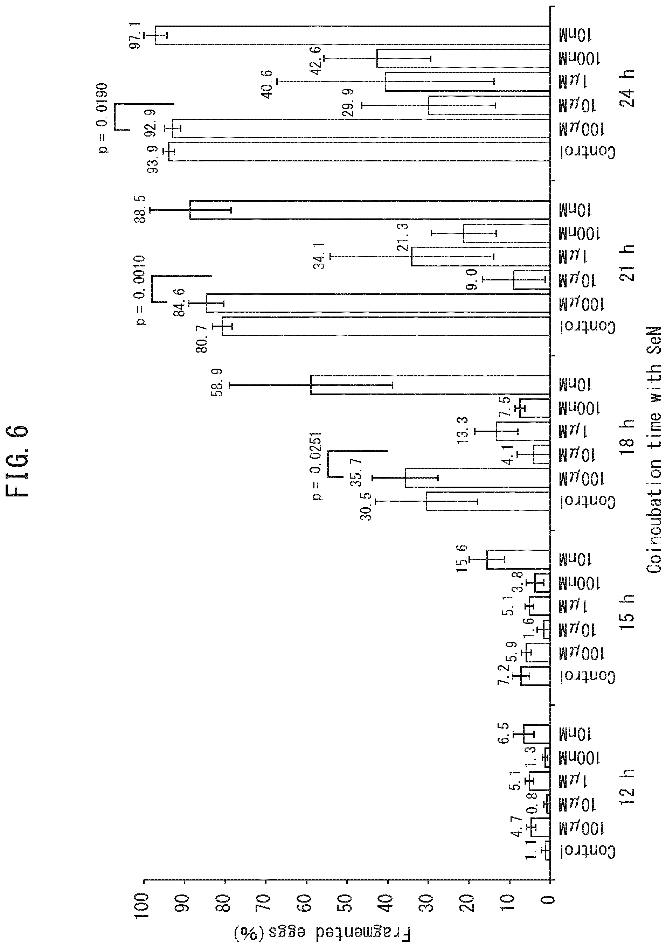

[Fig. 6] Fig. 6 shows the proportion of eggs exhibiting fragmentation at 12 hours, 15 hours, 18 hours, 21 hours and 24 hours after culturing eggs in TYH medium containing 10 nM, 100 nM, 1 µM, 10 µM and 100 µM selenoneine.

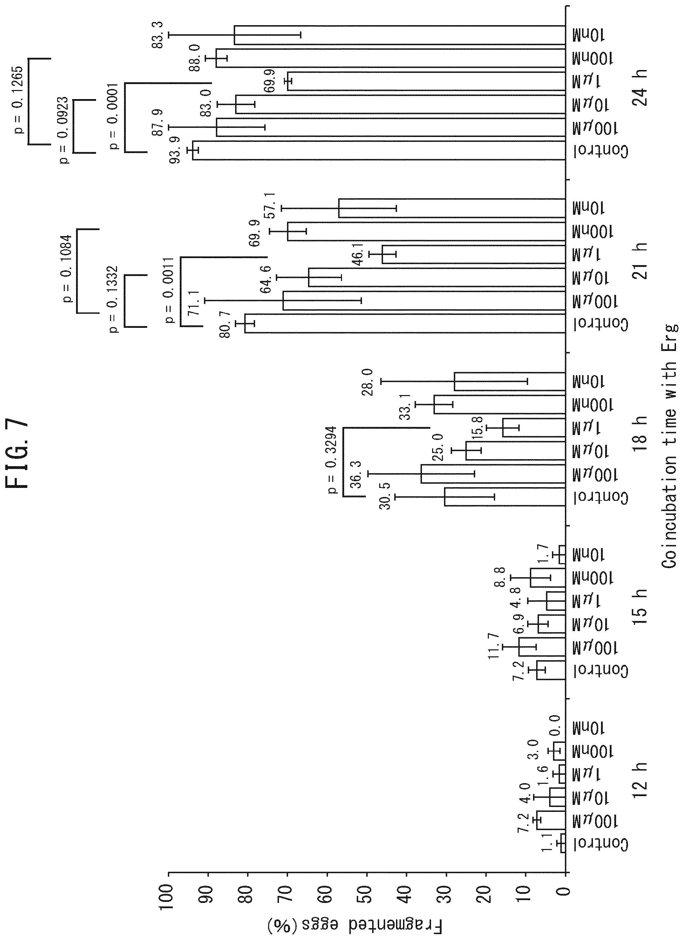

[Fig. 7] Fig. 7 shows the proportion of eggs exhibiting fragmentation at 12 hours, 15 hours, 18 hours, 21 hours and 24 hours after culturing eggs in TYH medium containing 10 nM, 100 nM, 1 µM, 10 µM and 100 µM ergothioneine.

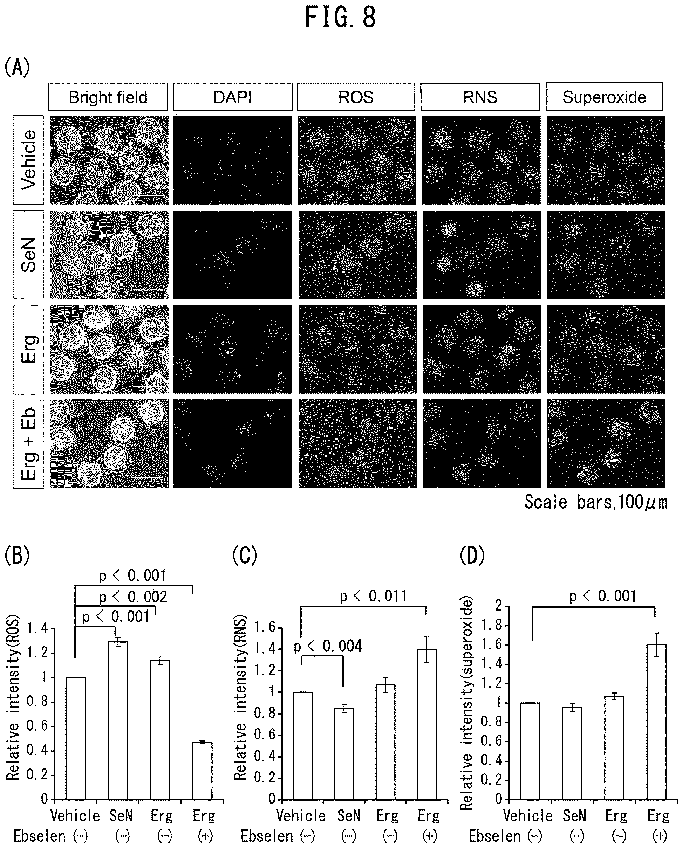

[Fig. 8] Fig. 8A is a fluorescent microscope photograph visualizing reactive oxygen species (ROS), reactive nitrogen species (RNS) and superoxide after culturing of eggs in a control with addition of DMSO alone (Vehicle), selenoneine-containing medium (SeN), ergothioneine-containing medium (Erg), and ergothioneine and ebselen-containing medium (Erg+Eb). Figs. 8B to D show relative amounts of reactive oxygen species (ROS), reactive nitrogen species (RNS) and superoxide in cells.

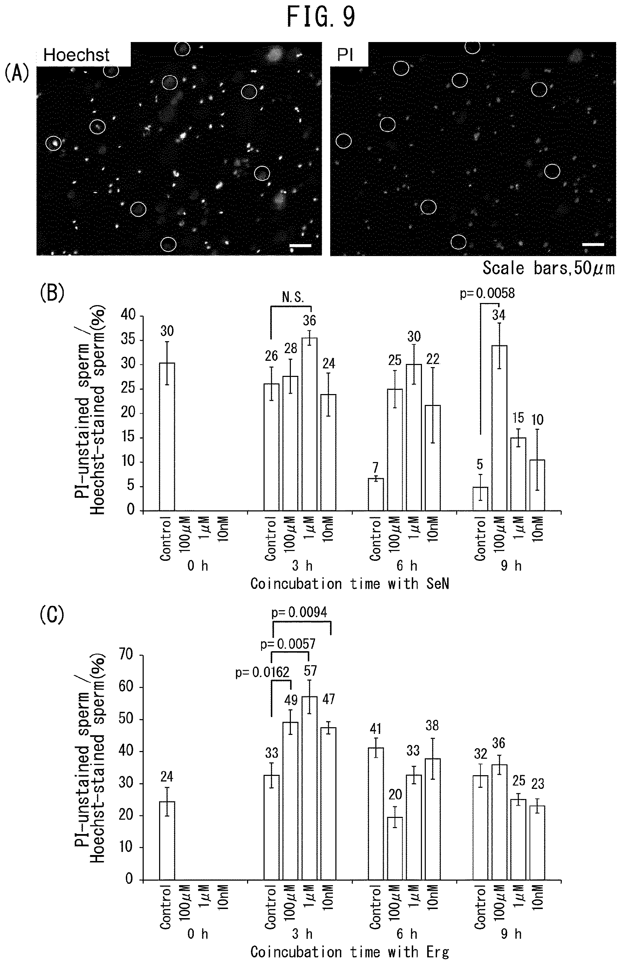

[Fig. 9] Fig. 9A shows a fluorescent microscope photograph of sperm nuclei stained with Hoechst and propidium iodide after culturing in selenoneine-containing medium. The circled sperm represent sperm stained with Hoechst, which were unstained with propidium iodide. Fig. 9B shows the proportion of the number of sperm stained with Hoechst AG and unstained with propidium iodide, divided by the number of sperm stained with Hoechst AG, among sperm cultured for 3 hours, 6 hours or 9 hours in TYH medium with a selenoneine concentration of 10 nM, 1 µM or 100 µM. Fig. 9C shows the number of sperm stained with Hoechst AG and unstained with propidium iodide, divided by the number of sperm stained with Hoechst AG, among sperm cultured for 3 hours, 6 hours or 9 hours in TYH medium with an ergothioneine concentration of 10 nM, 1 µM or 100 µM.

### DESCRIPTION OF EMBODIMENTS

Embodiments of the present invention (hereunder also referred to as "the embodiments") will be described in detail below, but the invention is not limited to the embodiments and may incorporate various modifications that do not deviate from the gist of the invention.

### (Agent for inhibiting Fragmentation)

- The agent for inhibiting fragmentation of fertilized egg according to the embodiment comprises a compound represented by the following formula (I): 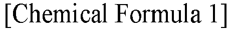 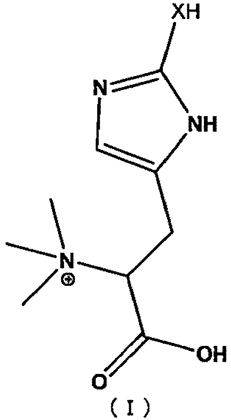 (wherein X is selenium (Se) or sulfur (S))
   or a pharmaceutically acceptable salt thereof, as an active ingredient. The OH group, XH group and NH group in the molecule may also lack the hydrogen atoms, i.e. they may be ionized. The compound may optionally be in the form of an optical isomer, geometric isomer, tautomer or dimer, or a mixture thereof. As a result of tautomerization and dimerization, the compound of the invention may be in the form of any of the following (I) to (III): 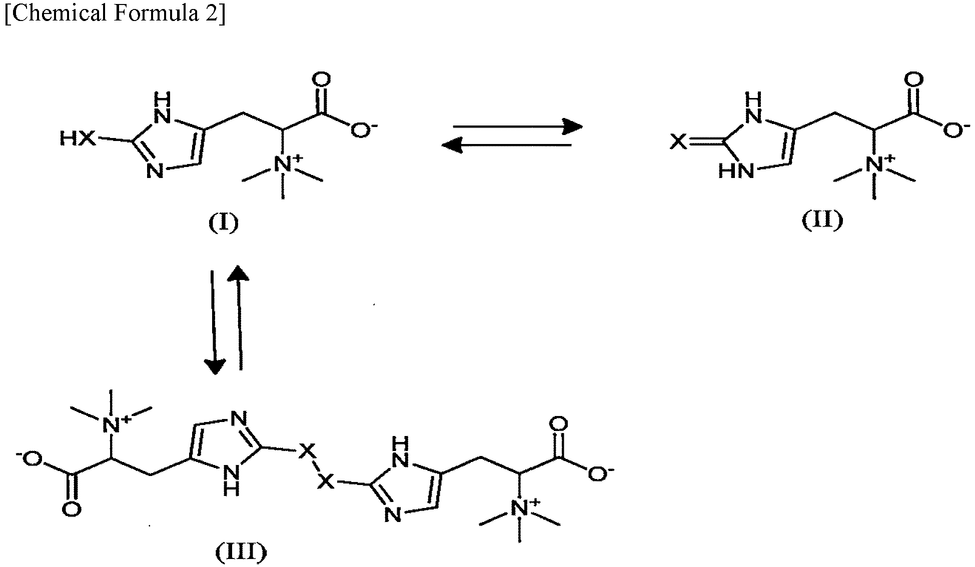 (wherein X is selenium (Se) or sulfur (S)). The agent for inhibiting fragmentation may comprise a compound in the form of any of formulas (I) to (III). The dimerized compound can be reduced to a monomer depending on the medium environment. A composition including a compound represented by formula (I) or (II) may further include a reducing agent so as to maintain the monomeric form. Any desired reducing agent, examples of which include glutathione (GSH), dithiothreitol (DTT) and mercaptoethanol. can be used as such a reducing agent. In the present specification, "an agent for inhibiting fragmentation" refers to an active ingredient, selenoneine or ergothioneine, or a tautomer or dimer thereof, or a pharmaceutically acceptable salt thereof.

According to one embodiment, the compound represented by formula (I) or (II) is a compound wherein X is a selenium atom, and such compounds will also be referred to herein as "selenoneine". According to another embodiment, compounds represented by formula (I) or (II) wherein X is a sulfur atom will also be referred to as "ergothioneine". As long as selenoneine or ergothioneine have the basic construction of formula (I) or (II), they may have the OH group, SeH group or NH group in the molecule in a state without the hydrogen atom, i.e. in the ionized state.

Selenoneine can be appropriately produced by a person skilled in the art. The method for producing the selenoneine may be a chemical synthesis method (Angew. Chem. Int. Ed. 2019, 58, 1-6), or selenoneine may be extracted from selenoneine-containing biological tissue, or produced by fermentation using microorganisms. Examples include a method of extraction from tissue of squid, fish, birds or mammals as described in Japanese Patent Publication No. 5669056; a method of using *Schizosaccharomyces pombe,* a fission yeast in which a gene associated with ergothioneine biosynthesis has been introduced described by Pluskal T et al. in (Pluskal T et al., PLoS One 2014 May 14; 9(5):e97774), and a method of producing histidine and selenium compounds using transformants of *Aspergillus* microorganisms such as *Aspergillus sojae, Aspergillus oryzae, Aspergillus niger,* or *Escherichia coli,* which overexpress a gene coding for selenoneine synthase, described in International Patent Publication No. WO2017/026173. For production of an agent for inhibiting fragmentation according to one embodiment of the invention on an industrial scale, the method described in International Patent Publication No. WO2017/026173 is preferred from the viewpoint of producing selenoneine at high yield.

In the method using a transformant that overexpresses selenoneine synthase described in WO2017/026173, ergothioneine is simultaneously produced with selenoneine, and because it is difficult to separate them, the transformant extract containing selenoneine may contain ergothioneine in addition to selenoneine. Selenoneine is preferably purified selenoneine if available. Selenoneine can be purified by a method known to those skilled in the art, such as HPLC.

Ergothioneine used may be a commercially available product, or it can be suitably produced by a person skilled in the art. Known methods for producing ergothioneine include chemical synthesis methods (Japanese Unexamined Patent Publication No. 2006-160748) and methods of extraction from organisms known to include ergothioneine (such as a basidiomycetes species such as Pleurotus citrinopileotus) (Japanese Patent Publication No. 4865083 and Japanese Patent Publication No. 5231025), as well as methods of extracting ergothioneine from microorganisms capable of producing ergothioneine (International Patent Publication No. WO2016/104437).

The term "fertilized egg" as used herein refers to an egg that has been fertilized, and it includes early embryos that have undergone cleavage. An early embryo refers to an embryo from the single-cell stage after fertilization up to the blastocyst stage, the embryo in the blastocyst stage optionally having been hatched. Early embryos that are in the two-cell stage to the 8-cell stage are referred to as "split embryos". An early embryo forms a morula after the 8-cell stage, subsequently forming a blastocyst. The fertilized egg may also be derived from a mammal such as a human. The mammal is preferably a human, but according to another embodiment the fertilized egg may be a fertilized egg taken from a non-human mammal such as a livestock animal, experimental animal, zoo animal or pet. Livestock animals are most preferably cows or pigs that have been treated by artificial insemination or *in vitro* fertilization.

The fertilized egg grows into an embryo by repeated division, but the term "inhibit fragmentation" is used herein to mean inhibiting formation of fragments during the growth process, and particularly the early embryo from the 2-cell stage to 8-cell stage. Fragmentation occurs when only the cytoplasm divides without undergoing normal nuclear fission. Fragmentation of the cytoplasm without the cell nucleus can be confirmed by bright field observation using a stereomicroscope. The degree of fragmentation is usually judged visually, and it can be judged as grade 1 to 5 according to the Veeck categorization, based on the level of fragmentation (Nakayama, T.: Rinsho Fujinka Sanka 2013:67(10), 1030-1037). The presence or absence of the cell nucleus can be confirmed visually or by fluorescent microscope observation, after adding DNA-binding fluorescent reagent (Hoechst 33342) to the medium and allowing the mixture to stand in an incubator at 37°C for 10 minutes.

The agent for inhibiting fragmentation may be used by addition to the medium used for *in vitro* fertilization, artificial insemination or intracytoplasmic sperm injection, or it may be intravaginally administered. The agent for inhibiting fragmentation may also be added to medium for gametes or fertilized eggs. According to another embodiment, the agent for inhibiting fragmentation may be added to medium for sperm. The sperm medium may be medium used for culturing until the sperm have acquired capacitation, or it may be medium used for refrigeration or cryopreservation. The sperm-fertilized or inseminated fertilized egg, wherein the sperm has been cultured in culture medium containing an agent for inhibiting fragmentation, can exhibit less fragmentation during the subsequent development process. If the sperm are cultured or stored in sperm medium containing the agent for inhibiting fragmentation and the prefertilization sperm are washed with medium, then it is possible to exclude infiltration of excess selenium into the egg. According to another embodiment, the agent for inhibiting fragmentation may be added to the egg and/or fertilized egg medium. The egg medium may be a medium for refrigeration or cryopreservation, or medium for fertilization. If the agent for inhibiting fragmentation is included in any one or more of the medium for egg storage, the medium for fertilization and the fertilized egg culture medium, then fragmentation can be inhibited during development of the early embryo.

The agent for inhibiting fragmentation of the invention can therefore be used as a sperm or egg treatment agent. Post-fertilization fragmentation is inhibited by treatment of sperm or eggs in medium containing the agent for inhibiting fragmentation. According to another embodiment, the agent for inhibiting fragmentation of the present invention can be considered to be an embryogenesis-promoting agent. By inhibiting fragmentation and promoting embryogenesis, it is possible to produce a split embryo, morula or blastocyst with a higher implantation rate.

Yet another embodiment relates to a sperm, egg, fertilized egg or early embryo medium containing an agent for inhibiting fragmentation of the invention. Such a medium may be medium for storage, or medium for culturing. Media for storage include cold storage and cryopreservation media. The sperm medium, egg medium, fertilized egg medium and early embryo medium may be the same or different. When medium exchange is carried out after fertilization, different medium may be used. The final concentration of selenoneine or ergothioneine added to each medium may be 1 nm to 10 µM. From the viewpoint of exhibiting an effect of inhibiting fragmentation, the final concentration is generally preferred to be 5 nM or greater, more preferably 10 nM or greater and even more preferably 50 nM or greater. From the viewpoint of avoiding toxicity, the final concentration is generally preferred to be 100 µM or lower, more preferably 50 µM or lower and even more preferably 20 µM or lower.

The sperm, egg, fertilized egg or early embryo medium may be any medium commonly obtainable or commonly used in the technical field, and the agent for inhibiting fragmentation of the invention may be added to the medium. Such media include, but are not limited to, TYH medium, HTF medium and CZB medium. Such media may also contain optional components commonly used in the technical field. The components in such media include electrolytes, polymers, energy source substances, pH adjustors and serum components, which may be selected as appropriate depending on the medium. Freezing medium may lack electrolytes from the viewpoint of preventing salt damage, and a cryoprotectant such as glycerol may be added from the viewpoint of preventing freeze injury.

Electrolytes include sodium chloride, potassium chloride, calcium chloride, magnesium chloride, potassium dihydrogen phosphate, dipotassium hydrogenphosphate, sodium hydrogencarbonate, sodium acetate and magnesium sulfate. An electrolyte is added for adjustment of the pH or osmotic pressure. Polymers include polyvinyl alcohol (PVA), polyvinylpyrrolidone (PVP) and Ficoll. Energy sources include glucose, lactic acid, pyruvic acid and their salts (such as sodium lactate or sodium pyruvate). Buffering agents such as HEPES, HES, TRIS and MES may be used as pH adjustors.

If necessary, an antibiotic such as penicillin, streptomycin or amphotericin B may be added to the fertilization medium. In addition, a pH indicator such as phenol red that allows change in medium pH to be visualized may be added.

The agent for inhibiting fragmentation may include either selenoneine or ergothioneine, or both, as an active ingredient. The agent for inhibiting fragmentation may also include other substances known to have effects of inhibiting fertilized egg fragmentation.

### (Sperm culturing method)

Another embodiment of the invention relates to a method of culturing sperm using medium containing the agent for inhibiting fragmentation of the invention. Specifically, the sperm culturing method comprises a step of culturing harvested sperm (unfrozen sperm) or frozen and thawed sperm in medium containing the agent for inhibiting fragmentation. Selenoneine is especially preferred as the agent for inhibiting fragmentation when frozen and thawed sperm are used. As an example of the sperm culturing method, droplets of culture medium are formed on a dish, mineral oil is laid over them, and harvested sperm are added to and cultured in medium placed in a 5% carbon dioxide atmosphere at 37°C. Culturing for 30 minutes to several hours usually causes hyperactivation of the sperm. The cultured sperm may then be used for artificial insemination or *in vitro* fertilization. For cryopreservation, the harvested sperm are transferred to freezing medium and dispensed into a container such as a cryopreservation straw to allow freezing in liquid nitrogen. Before cryopreservation, the sperm may be cultured in medium containing an agent for inhibiting fragmentation, or the agent for inhibiting fragmentation may be included in the freezing medium for freezing, or the agent for inhibiting fragmentation may be included in the washing solution, thawing solution or culture medium after freezing and thawing, prior to washing or culturing.

### (Egg culturing method)

Another embodiment of the invention relates to a method of culturing an egg using medium containing the agent for inhibiting fragmentation of the invention. Specifically, the egg culturing method includes a step of culturing a harvested egg (unfrozen egg) or a frozen and thawed egg in medium containing the agent for inhibiting fragmentation. As an example of the egg culturing method, droplets of culture medium are formed on a dish, mineral oil is laid over them, and the harvested egg is added to and cultured in medium placed in a 5% carbon dioxide atmosphere at 37°C. The culture medium containing the egg is used for *in vitro* fertilization (IVF) by addition of culture medium containing sperm. For cryopreservation, the harvested egg is transferred to freezing medium and dispensed into a cryopreservation straw or wire mesh to allow freezing in liquid nitrogen. Before cryopreservation, the egg may be cultured in medium containing an agent for inhibiting fragmentation, or the agent for inhibiting fragmentation may be included in the freezing medium for freezing, or the agent for inhibiting fragmentation may be included in the washing solution, thawing solution or culture medium after freezing and thawing, prior to washing or culturing.

### (Fertilization method)

Another embodiment of the invention relates to a fertilization method using medium containing the agent for inhibiting fragmentation of the invention. In the fertilization method of the invention, fertilization is carried out using medium containing the agent for inhibiting fragmentation. More specifically, the agent for inhibiting fragmentation may be included in medium in which the egg has been placed, or the agent for inhibiting fragmentation may be included in medium in which sperm have been cultured. The agent for inhibiting fragmentation is preferably included in both the medium in which the egg has been placed and the medium in which the sperm have been cultured. The fertilization method includes a step of mixing the medium in which the egg has been placed with the sperm-containing medium. In a different method, sperm in a good condition may be selected out from the sperm-containing medium and used to fertilize the egg by intracytoplasmic sperm injection. The fertilized egg may be cultured directly in medium or cultured after exchanging the medium as the embryo develops. The agent for inhibiting fragmentation may also be included in the exchanged medium. The fertilized egg may also be transferred into the freezing medium and dispensed into a cryopreservation straw or wire mesh to allow freezing in liquid nitrogen. Before cryopreservation, the egg may be cultured in medium containing an agent for inhibiting fragmentation, or the agent for inhibiting fragmentation may be included in the freezing medium for freezing, or the agent for inhibiting fragmentation may be included in the washing solution, thawing solution or culture medium after freezing and thawing, prior to washing or culturing.

### (Method of culturing fertilized egg or embryo)

After fertilization, the fertilized egg may be cultured in the same medium or in a medium subjected to medium exchange. Culturing is preferably carried out in medium containing the agent for inhibiting fragmentation. The fertilized egg or embryo may be unfrozen, or used after freezing and thawing. The fertilized egg, which is obtained immediately after fertilization, or the cryopreserved fertilized egg can be used in the method of culturing the fertilized egg. The fertilized egg forms a morula via the 2-cell stage, 4-cell stage and 8-cell stage, over several days after fertilization, and subsequently forms a blastocyst. The method of culturing the fertilized egg or embryo may therefore be a method for producing a split embryo, morula or blastocyst, by being combined with the fertilization method. The split embryo, morula or blastocyst produced by the method may then be returned to the uterus.

### (Infertility improver)

The present invention relates to an agent for improving infertility comprising selenoneine or ergothioneine, or a tautomer or dimer thereof, or a pharmaceutically acceptable salt thereof. Ergothioneine is not formed in the mammalian body but is orally ingested, and it is known to accumulate in specific organs. Ergothioneine is known to be distributed in seminal vesicles together with ergothioneine transporter, and is also known to be present in seminal fluid secreted from the seminal vesicles (J. Physiology and Pharmacology (2011), 62, 4, 411-419). In birds, providing an ergothioneine-containing diet is known to result in its accumulation in eggs (International Patent Publication No. 2015/098380). Selenoneine is a serine analog of ergothioneine and is therefore thought to be similarly distributed in the body. Selenoneine and ergothioneine can accumulate in seminal vesicles and ovaries by ingestion and are present in seminal fluid secreted by the seminal vesicles, having a fragmentation-inhibiting effect when acting on sperm and eggs. Therefore, selenoneine or ergothioneine, or a tautomer or dimer thereof, or a pharmaceutically acceptable salt thereof, can be used as an agent for inhibiting infertility, and can be formulated for oral dosage. The agent for improving infertility of the invention can be provided as an agent for improving infertility for males or for females. The agent for improving infertility of the invention can be sold as a drug, a quasi drug or a functional food. Examples of functional foods include supplements, fortified foods and fortified beverages. For use as an ingestible agent for improving infertility, selenoneine or ergothioneine, or a tautomer or dimer thereof, or a pharmaceutically acceptable salt thereof, can be administered at 0.1 to 100 mg, for example, as a daily dose.

### (Infertility treatment method)

Infertility treatment methods generally include artificial insemination, *in vitro* fertilization and intracytoplasmic sperm injection. These treatment methods include culturing of harvested sperm or eggs, or fertilized eggs, in medium containing an agent for inhibiting fragmentation. The specific steps used in infertility treatment methods are described in Fertility and Sterility (2017), vol. 107, No. 4 pp.882-896, for example. According to yet another aspect, it is possible to administer an agent for inhibiting fragmentation, or selenoneine or ergothioneine, or a tautomer or dimer thereof, or a pharmaceutically acceptable salt thereof, for infertility treatment. The route of administration used may be any desired route such as oral administration, for example. The administered individuals may be a female and partner desiring pregnancy. Administration may also be to a male suffering from male infertility such as spermatogenesis deficiency.

The present invention will now be further described by Examples, with the understanding that the invention is not limited by the Examples and may incorporate various modifications so long as the problem of the invention can be solved. All of the publications mentioned throughout the present specification are incorporated herein in their entirety by reference.

### EXAMPLES

### Example 1. Preparation of selenoneine

### Preparation of selenoneine-containing filamentous fungi extract and ergothioneine-containing filamentous fungi extract

*Aspergillus sojae* transformants that had been transformed with the AsEgtA gene described in WO 2017/026173 were used to obtain selenoneine-containing filamentous fungi extracts by the method described in Example 7.

### Purification of selenoneine

### (1) Preparation of test sample and reference sample

A portion of the selenoneine-containing filamentous fungi extract obtained in Example 1 (selenoneine concentration: 136.8 µg/mL) was provided for freeze-drying and a small amount was dissolved in water for concentration.

The concentration sample was fractionated under the following HPLC conditions.
Device: LC-8A pump, UV/VIS detector SPD-20A, fraction collector FRC-10A (product of Shimadzu Corp.).
Column: Develosil RPAQUEOUS-AR-5 20 × 250 mm (Nomura Chemical Co., Ltd.)
Eluent: 5 mM ammonium acetate
Flow rate: 5 mL/min
Detection: 220 nm
Fractionation: 1.25 mL each

A portion of selenoneine obtained by the process of fractionation by preparative HPLC, freeze-drying and dissolution in 80% ethanol was dried with a centrifugal evaporator and then dissolved in water. After confirming the concentration by LC-ICP-MS it was adjusted to 10 mM (2.77 mg/mL) with water.

Fig. 1 shows the results of analytical HPLC of the 80% ethanol supernatant fraction obtained after purification by preparative HPLC.

### Example 2. Sperm motility test

TYH medium was prepared with the following composition.

**[Table 1]**

| TYH medium | |
|---|---|
| Composition | (mg/500 ml) |
| NaCl | 3,488 |
| KCl | 178 |
| CaCl₂ | 95 |
| KH₂PO₄ | 81 |
| MgSO₄ ·7H₂O | 146.5 |
| NaHCO₃ | 1,053 |
| Glucose | 500 |
| Sodium pyruvate | 27.5 |
| Penicillin G | 37.5 |
| Streptomycin | 25 |
| Bovine serum albumin | 2,000 |
| 1% Phenol Red | 0.5 (ml) |

A 100 µL drop of TYH medium was prepared in a plastic dish, and liquid paraffin was laid over it until the drop was completely covered. The plastic dish was then allowed to stand for 16 hours in an incubator set to 5% CO₂, 37°C. The TYH medium drop on the plastic dish was used for a sperm motility test and fertilization test.

A sperm mass harvested from the epididymis of a 8- to 12-week-old sexually matured male mouse (C57BL/6 J line) was transferred to a drop prepared in the TYH medium. It was then returned to the plastic dish in the incubator and allowed to stand for 1 hour. After standing for 1 hour, 1 µL of selenoneine (10 µM) prepared in Example 1 was added to a drop of sperm-containing fertilization medium (or not added for the control), and the mixture was allowed to stand in an incubator. After incubation for 3 hours, the sperm motility was measured using CASA, and the proportion of sperm was measured, dividing it into motile sperm and progressive sperm (Figs. 2A and B). After incubation for 6 hours, the proportion of sperm was then measured with similar division based on sperm motility (VCL) (Figs. 2C and D). No effect on sperm motility was seen between the control group and the selenoneine-added group.

### Example 3: Fertilized egg fragmentation-inhibiting effect by selenoneine treatment

Female mice (C57BL/6 J line: 8- to 12-week-old) were treated for superovulation by the following procedure, and the eggs were harvested.
1. Animal serotropin (PMSG, ASKA Pharmaceutical Co., Ltd.) and animal gonadotropin (hCG, ASKA Pharmaceutical Co., Ltd.) were dissolved in physiological saline to 37.5 unit/mL.
2. The mice were intraperitoneally administered PMSG at 0.1 mL(5 units)/mouse, and after 48 hours they were intraperitoneally administered hCG at 0.1 mL (5 units)/mouse.
3. After hCG administration, the cumulus cells and egg masses were taken through the fallopian tubes of the mice at 14 to 16 hours.

The cumulus cells and egg masses harvested from the fallopian tubes of the superovulated mice were transferred to a drop of 100 µL TYH medium that had been previously incubated in 5% CO₂ at 37°C, and allowed to stand in the incubator.

### Selenoneine treatment of eggs

The harvested eggs were cultured for 2 hours in TYH medium, and then the 1 µL selenoneine (10 µM) solution prepared in Example 1 was added to the TYH medium drop (or not added for the control), and further culturing was carried out for 2 hours with a final concentration of 100 nM selenoneine in the medium. After culturing, 10 µL of the sperm incubated for 6 hours in non-added TYH medium for the control of Example 2 was separated off together with medium, and added to the TYH medium containing cumulus cells-egg masses. After 24 hours had passed in the presence of selenoneine, the fertilization rate was measured and the numbers of normal 2-cell stage embryos and fragmented embryos were counted. For measurement of the fertilization rate, second polar body release and the presence or absence of cell nuclei in the split bodies of each of the 2-cell stage embryos were visually observed in the bright field of a stereomicroscope, and normal 2-cell stage embryos and fragmented embryos were likewise observed in the bright field of a stereomicroscope. Fig. 3A shows a photograph in a bright field. While there was no difference in the proportion of 2-cell stage embryos (Fig. 3A), the eggs treated with selenoneine showed no fragmented embryos, indicating inhibition of fragmentation by selenoneine (Fig. 3B).

### Selenoneine treatment of sperm

In the same manner as Example 2, sperm incubated for 6 hours in selenoneine-containing TYH medium (final concentration: 100 nM) were separated off with the medium and washed with TYH medium that had been previously incubated at 37°C in a 5% CO₂ atmosphere, after which the washed sperm were separated off and added to TYH medium containing cumulus cells-egg masses. After 24 hours had passed, the fertilization rate was measured and the numbers of normal 2-cell stage embryos and fragmented embryos were counted. For measurement of the fertilization rate, second polar body release and the presence or absence of cell nuclei in the split bodies of each of the 2-cell stage embryos were visually observed in the bright field of a stereomicroscope, and normal 2-cell stage embryos and fragmented embryos were likewise observed in the bright field of a stereomicroscope. The measurement results are shown in Fig. 4. While there was no difference in the proportion of 2-cell stage embryos (Fig. 4A), the eggs treated with selenoneine showed no fragmented embryos, indicating inhibition of fragmentation by selenoneine (Fig. 4B).

### Example 4: Fragmentation-inhibiting effect by ergothioneine treatment

### Ergothioneine treatment and selenoneine treatment of sperm

The inhibiting effect of ergothioneine treatment on fragmentation of fertilized eggs was examined in the same manner as the selenoneine treatment of sperm in Example 3, but using commercially available ergothioneine (final concentration: 100 nM) instead of selenoneine (Fig. 5). No difference in the proportion of 2-cell stage embryos was found between the ergothioneine treatment and selenoneine treatment (Fig. 5A). No fragmented embryos were present with selenoneine treatment, and the proportion of fragmented embryos with ergothioneine treatment was reduced compared to the untreated control (Fig. 5B).

### Example 5: Fragmentation-inhibiting effect of selenoneine and ergothioneine for fragmentation induced by stress

### Selenoneine treatment of eggs

Mouse cumulus cells-egg masses harvested by the method of Example 3 were stored in TYH medium and the cumulus cells were removed by 300 µg/mL hyaluronidase treatment to recover the eggs alone. TYH medium was prepared with selenoneine at 10 nM, 100 nM, 1 µM, 10 µM and 100 µM, and incubated on a hot plate set to 37°C. Selenoneine-free THY medium was used as the control. The collected eggs were transferred to the incubated selenoneine-containing TYH medium and cultured under shielding for 12 hours, 15 hours, 18 hours, 21 hours and 24 hours, after which they were observed under a stereomicroscope and the proportion of fragmented eggs was examined (Fig. 6). The numbers of cultured eggs in each selenoneine-containing medium were 10 nM: 107, 100 nM: 161, 1 µM: 155, 10 µM: 118, 100 µM: 85 and control: 83.

Culturing by incubation on a hot plate instead of a CO₂ incubator produced stress in the eggs. At 18 hours after culturing on the hot plate, the proportion of eggs exhibiting fragmentation increased. With culturing in TYH medium containing selenoneine at concentrations of 100 nM, 1 µM and 10 µM, on the other hand, the degree of egg fragmentation was significantly inhibited (p < 0.05). The numbers of cultured eggs in each medium were 10 nM: 63, 100 nM: 66, 1 µM: 63, 10 µM: 71, 100 µM: 54 and control: 83.

### Ergothioneine treatment of eggs

Mouse cumulus cells-egg masses harvested by the method of Example 3 were stored in TYH medium and the cumulus cells were removed by 300 µg/mL hyaluronidase treatment to recover the eggs alone. TYH medium was prepared with ergothioneine at 10 nM, 100 nM, 1 µM, 10 µM and 100 µM, and incubated on a hot plate set to 37°C. Ergothioneine-free THY medium was used as the control. The collected eggs were transferred to the incubated selenoneine-containing TYH medium and cultured under shielding for 12 hours, 15 hours, 18 hours, 21 hours and 24 hours, after which they were observed under a stereomicroscope and the proportion of fragmented eggs was examined (Fig. 7).

Culturing by incubation on a hot plate instead of a CO₂ incubator produced stress in the eggs. At 18 hours after culturing on the hot plate, the proportion of eggs exhibiting fragmentation increased. After 21 hours of culturing and 24 hours of culturing, culturing in TYH medium containing ergothioneine at 1 µM concentration significantly inhibited egg fragmentation (p < 0.05), with inhibition seen when using concentrations of 10 nM to 10 µM after 21 hours of culturing and 24 hours of culturing.

### Example 6: Oxidative stress test

Mouse cumulus cells-egg masses harvested by the method of Example 3 were stored in TYH medium and the cumulus cells were removed by 300 µg/mL hyaluronidase treatment to recover the eggs alone. TYH medium containing 1 µM selenoneine (SeN), TYH medium containing 1 µM ergothioneine (Erg) and TYH medium containing 1 µM ergothioneine and 1 µM ebselen (Tokyo Kasei Kogyo Co., Ltd.) (Erg+Eb) were prepared and incubated on a hot plate set to 37°C. As a control, a TYH medium (vehicle) containing the DMSO (0.1%) used to dissolve the ebselen was used. The collected eggs were transferred to incubated selenoneine-containing TYH medium and cultured under shielding for 15 hours. The reagent of a ReOIS-ID ROS/RNS Detection Kit (product of Enzo Co.) was added to the medium containing the cultured eggs, according to the manufacturer's protocol. DAPI (Wako Pure Chemical Industries, Ltd.) had already been separately added to stain the nuclei. The nuclei (DAPI) and the reactive oxygen species (ROS), reactive nitrogen species (RNS) and superoxides in the eggs were observed under a fluorescent microscope (Fig. 8A). The fluorescence intensity was calculated and graphed (Figs. 8B to D).

The reactive oxygen species increased significantly with medium containing added selenoneine and ergothioneine, but active oxygen decreased in medium containing added ebselen as an antioxidant (Erg+Eb). A significant reduction in reactive nitrogen species was seen with addition of selenoneine, but addition of ergothioneine showed no variation, and the reactive nitrogen species increased in medium containing added ebselen as an antioxidant (Erg+Eb). No change was seen in superoxides with selenoneine addition or ergothioneine addition, but they significantly increased in medium containing added ebselen as an antioxidant (Erg+Eb). To summarize, consistent results were not obtained for the amounts of reactive oxygen species, reactive nitrogen species and superoxides, and no connection was found between selenoneine and ergothioneine, and oxidative stress.

### Example 7: Protective effect of selenoneine and ergothioneine treatment on sperm

### Selenoneine treatment of sperm

A sperm mass harvested from the epididymis of an 8- to 12-week-old sexually matured male mouse (C57BL/6 J line) was transferred to a drop prepared in the TYH medium. It was then returned to the plastic dish in the incubator and allowed to stand for 1 hour. After standing for 1 hour, the selenoneine prepared in Example 1 was added to a drop of sperm-containing fertilization medium to final concentrations of 10 nM, 1 µM and 100 µM (or was not added for the control), and the mixture was cultured in an incubator for 3 hours, 6 hours and 9 hours, after which the sperm were obtained. The sperm were also obtained before culturing (0 h). The nuclei of obtained sperm were stained with 2 µg/mL of propidium iodide (PI: Sigma-Aldrich Japan, KK.) and 16.2 µM of Hoechst AG 33342 (Hoechst3342: Invitrogen Corp.), and observed under a fluorescent microscope (Fig. 9A). The number of sperm whose nuclei were unstained with propidium iodide was divided by the number of sperm whose nuclei were stained with Hoechst AG, to calculate the proportion of sperm which retained membranes (Fig. 9B).

Hoechst AG is cell membrane-permeable and can stain all sperm nuclei, whereas propidium iodide is membrane-impermeable and can only stain the nuclei of dead sperm or sperm that have damaged membranes. Therefore, the number of sperm whose nuclei were not stained with propidium iodide but were stained only by Hoechst AG represents the number of normal sperm which retained their membranes. Continued culturing resulted in a lower proportion of sperm with retained membranes. Addition of selenoneine at a concentration of 10 nM to 100 µM exhibited a membrane-protecting effect for sperm, and the sperm membranes were significantly protected with a concentration of 100 µM even after culturing for 9 hours (p < 0.05).

### Ergothioneine treatment of sperm

A sperm mass harvested from the epididymis of an 8- to 12-week-old sexually matured male mouse (C57BL/6 J line) was transferred to a drop prepared in the TYH medium. It was then returned to the plastic dish in the incubator and allowed to stand for 1 hour. After standing for 1 hour, the selenoneine prepared in Example 1 was added to a drop of sperm-containing fertilization medium to final concentrations of 10 nM, 1 µM and 100 µM (or was not added for the control), and the mixture was cultured in an incubator for 3 hours, 6 hours and 9 hours, after which the sperm were obtained. The sperm were also obtained before culturing (0 h). The nuclei of obtained sperm were stained with 2 µg/mL of propidium iodide (PI: Sigma-Aldrich Japan, KK.) and 16.2 µM of Hoechst AG 33342 (Hoechst3342: distributed by Invitrogen Corp.), and observed under a fluorescent microscope. The number of sperm whose nuclei were unstained with propidium iodide was divided by the number of sperm whose nuclei were stained with Hoechst AG, to calculate the proportion of sperm which retained membranes (Fig. 9C).

Hoechst AG is cell membrane-permeable and can stain all sperm nuclei, whereas propidium iodide is membrane-impermeable and can only stain the nuclei of dead sperm or sperm that have damaged membranes. Therefore, the number of sperm whose nuclei were not stained with propidium iodide but were stained only by Hoechst AG represents the number of normal sperm which retained their membranes. Continued culturing resulted in a lower proportion of sperm with retained membranes. Addition of ergothioneine at a concentration of 10 nM to 100 µM exhibited a membrane-protecting effect for sperm, and the sperm membranes were significantly protected with a concentration of 100 µM even after culturing for 9 hours (p < 0.05).

### INDUSTRIAL APPLICABILITY

Since the agent for inhibiting fragmentation as one aspect of the invention can inhibit fragmentation or destruction of eggs and fertilized eggs, and can protect sperm cell membranes, by its active ingredient selenoneine or ergothioneine, it is thought to be useful for improvement of infertility treatment. More specifically, it is expected to increase implantation rates and pregnancy rates in infertility treatments such as artificial insemination and *in vitro* fertilization. It is also expected to increase implantation rates and pregnancy rates even in fields such as livestock in which artificial insemination and *in vitro* fertilization are commonly carried out.

## Claims

1. An agent for inhibiting fragmentation of fertilized egg, comprising selenoneine or ergothioneine, or a tautomer or dimer thereof, or a pharmaceutically acceptable salt thereof.

2. A sperm or egg treatment agent, comprising the agent for inhibiting fragmentation according to claim 1.

3. An embryogenesis promoting agent, comprising the agent for inhibiting fragmentation according to claim 1.

4. A medium for sperm culturing, egg culturing, fertilized egg culturing or fertilization, comprising the agent for inhibiting fragmentation according to claim 1.

5. A medium for freezing of sperm, eggs or fertilized eggs, comprising the agent for inhibiting fragmentation according to claim 1.

6. A sperm culturing method, comprising culturing sperm in medium that includes the agent for inhibiting fragmentation according to claim 1.

7. An egg or fertilized egg culturing method comprising culturing an egg or fertilized egg in medium that includes the agent for inhibiting fragmentation according to claim 1.

8. A method for producing a split embryo, morula or blastocyst, comprising the following steps:
culturing an egg in medium that includes the agent for inhibiting fragmentation according to claim 1,
fertilizing the cultured egg with sperm, and
culturing the fertilized egg in fertilized egg culturing medium.

9. A method for producing a split embryo, morula or blastocyst, comprising the following steps:
culturing sperm in medium that includes the agent for inhibiting fragmentation according to claim 1,
using the cultured sperm to fertilize an egg, and
culturing the fertilized egg in fertilized egg culturing medium.

10. The method according to claim 8 or 9, which further comprises a step of freezing a split embryo, morula or blastocyst by liquid nitrogenization.

11. The method according to any one of claims 8 to 10, wherein the step of fertilization is carried out by *in vitro* fertilization or intracytoplasmic sperm injection.

12. The method according to any one of claims 8 to 11, wherein the fertilized egg culturing medium further includes the agent for inhibiting fragmentation.

13. An agent for improving infertility comprising selenoneine or ergothioneine.
